# EUROPEAN PATENT SPECIFICATION

(11) **EP 2 675 454 B1**
(45) Date of publication and mention of the grant of the patent: **22.01.2020**
(21) Application number: 12704489.9
(22) Date of filing: 08.02.2012
(51) Int. Cl.: A61K 31/4468, A61K 9/00, A61K 47/10, A61K 47/14

(54) **METHODS FOR CONTROLLING PAIN IN EQUINES USING A TRANSDERMAL SOLUTION OF FENTANYL**
VERFAHREN ZUR STEUERUNG VON SCHMERZEN BEI PFERDEN UNTER VERWENDUNG EINER TRANSDERMALEN FENTANYLLÖSUNG
MÉTHODES DE RÉGULATION DE LA DOULEUR CHEZ LES ÉQUIDÉS AU MOYEN D'UNE SOLUTION TRANSDERMIQUE DE FENTANYL

(30) Priority: 15.02.2011 US 201161442878 P
(43) Date of publication of application: 25.12.2013
(73) Proprietor: Audevard, 92110 Clichy (FR)
(72) Inventor: MARR, Amy Louise, Indianapolis, IN 46206-6288 (US); MOUZIN, Douglas Eugene, Indianapolis, IN 46206-6288 (US); OWENS, Jane Granville, Indianapolis, IN 46206-6288 (US); RIGGS, Kari Lynette, Indianapolis, IN 46206-6288 (US)
(74) Representative: Regimbeau
(86) International application number: PCT/US2012/024252
(87) International publication number: WO 2012/112355

(56) References cited:
- WO-A1-2004/000263
- WO-A1-2004/000275
- WO-A1-2005/051476
- WO-A1-2011/049951
- WO-A2-2009/047779
- US-A1- 2004 028 625
- J. A. Orsini ET AL: "Pharmacokinetics of fentanyl delivered transdermally in healthy adult horses ? variability among horses and its clinical implications", Journal of Veterinary Pharmacology and Therapeutics, vol. 29, no. 6, 1 December 2006 (2006-12-01), pages 539-546, XP55140949, ISSN: 0140-7783, DOI: 10.1111/j.1365-2885.2006.00796.x
- LEHMANN K A ET AL: "Transdermal fentanyl: Clinical pharmacology", JOURNAL OF PAIN AND SYMPTOM MANAGEMENT, ELSEVIER, NEW YORK, NY, US, vol. 7, no. 3, 1 April 1992 (1992-04-01), pages S8-S16, XP023115377, ISSN: 0885-3924, DOI: 10.1016/0885-3924(92)90048-M [retrieved on 1992-04-01]

## Description

Opioids are an important part of multi-modal perioperative analgesia, especially for moderate to severe pain. In human health, the use of opioids during and after surgery for most soft tissue and orthopedic surgeries is considered as a standard of care and are included in procedure specific treatment algorithms. In veterinary medicine, the use of opioids is limited by undesirable pharmacokinetic parameters in many animals, including poor oral bioavailability and rapid clearance of opioids.

Fentanyl is a potent, full µ-opioid receptor agonist having approximately 100-fold the analgesic properties of the opioid morphine. However, poor oral bioavailability and rapid clearance has limited the use of fentanyl in many animals. Furthermore, the use of fentanyl in animals such as equines may be limited by dose dependent effects of central nervous system (CNS) excitation, gastrointestinal stasis, and increased locomotor activity following administration.

Various methods to deliver fentanyl to animals have been attempted in order to overcome these limitations and to prolong the therapeutic duration of action. For example, methods of transdermal application such as patches have potential advantages over oral and parenteral administration, including non-invasive dosing, avoidance of the gastrointestinal tract, lack of first pass metabolism, steady, continuous drug delivery rather than a peak and trough phenomenon, potential reduction of side effects by elimination of peaks, possible reduction of lack of effectiveness due to elimination of troughs, and reduced dose frequency for convenience and increased compliance. As an example, transdermal delivery of fentanyl in a patch formulation has been used in canines and in equines.

However, the use of fentanyl patches in equines introduces numerous additional shortcomings, including the lack of regulatory approval in equines, the slow delivery rate of fentanyl to equines, the low extent of fentanyl delivery in equines, the possibility of damage to patches applied to equines, the possibility of consumption of patches applied to equines and subsequent toxicity to the equines following consumption, the concern for proper control and disposal of used patches, the possibility of diversion and illicit use of patches, and the lack of regulatory oversight and pharmacovigilance to track adverse events in equines.

Therefore, there exists a need for a method to use fentanyl that overcomes some or all of the limitations of parenterally-, orally-, or patch-delivered opioids in order to benefit pain management in veterinary medicine. Accordingly, the present invention provides methods of using a transdermal pharmaceutical solution formulation of fentanyl which exhibits desirable properties and provides related advantages for the control of pain in equines. US 2004/028625 and J.A. Orsini et al, Journal of Veterinary Pharmacology and Therapeutics, Vol. 29, no. 6, 01 December 2006, pages 539-546 describe the transdermal delivery of analgesics.

The present invention demonstrates that the dermal barrier to drug permeation can be overcome in equines by using a transdermal pharmaceutical formulation according to the claims comprising fentanyl, a penetration enhancer, and a volatile liquid. Through deposition of fentanyl in the stratum corneum of an equine followed by prolonged systemic absorption, the present invention overcomes the limitations of poor oral bioavailability as well as the short duration of action of orally and parenterally administered fentanyl.

### BRIEF DESCRIPTION OF DRAWINGS

The teachings of some embodiments of the present invention will be better understood by reference to the description taken in conjunction with the accompanying drawings, wherein:
FIG. 1 is a perspective view of an assembled applicator connected to a fluid delivery device and positioned on an animal for dispensing a formulation onto the animal. In this perspective, a canine is used for illustrative purposes. In accordance with some embodiments of the present invention, the delivery device is used for dispensing a formulation onto an equine.
FIG. 2 is a perspective view an assembled applicator in accordance with some embodiments;
FIG. 2A is an end view of an outlet from the assembled applicator of FIG. 2 taken along line 2A;
FIG. 3 is a perspective view of the bottom section of an applicator in accordance with some embodiments;
FIG. 4 is another perspective view of the bottom section of an applicator in accordance with some embodiments;
FIG. 4A is a cross-sectional view of the top section of an applicator of FIG. 5 taken along line 5A;
FIG. 4B is a cross-sectional view of the bottom section of an applicator of FIG. 4 taken along line 4B;
FIG. 4C is a cross-sectional view of the assembled applicator of FIG. 2 taken along line 4C;
FIG. 5 is a perspective view of the top section of an applicator in accordance with some embodiments;
FIG. 5A is a cross-sectional view of a different embodiment of a top section of an applicator;
FIG. 5B is a cross-sectional view of a different embodiment of a bottom section of an applicator;
FIG. 5C is a cross-sectional view of the assembled applicator after the top section of FIG. 5A is ultrasonically welded with the bottom section of FIG. 5B;
FIG. 6 is a cross-sectional side view of an assembled applicator of FIG. 2 taken along line 6;
FIG. 7A is a cross-sectional view of another embodiment of a top section of an applicator;
FIG. 7B is a cross-sectional view of another embodiment of a bottom section of an applicator;
FIG. 7C is a cross-sectional view of the assembled applicator after the top section of

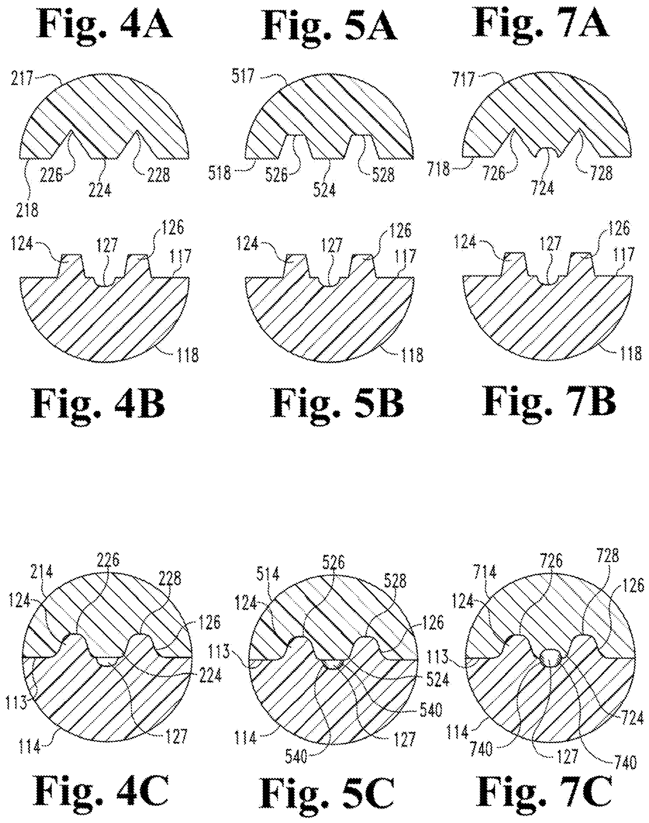

FIG. 7A is ultrasonically welded with the bottom section of FIG. 7B;

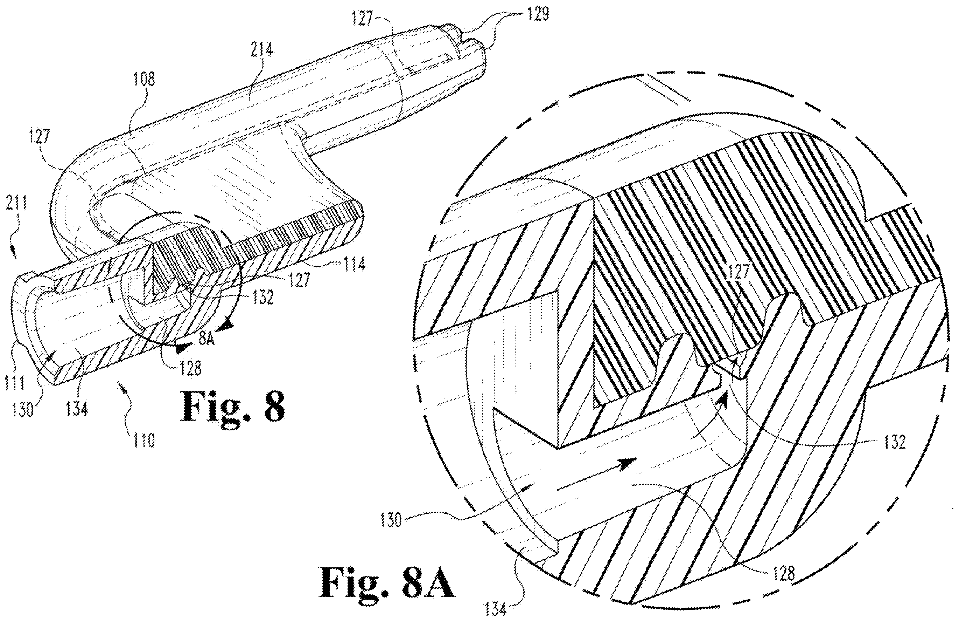

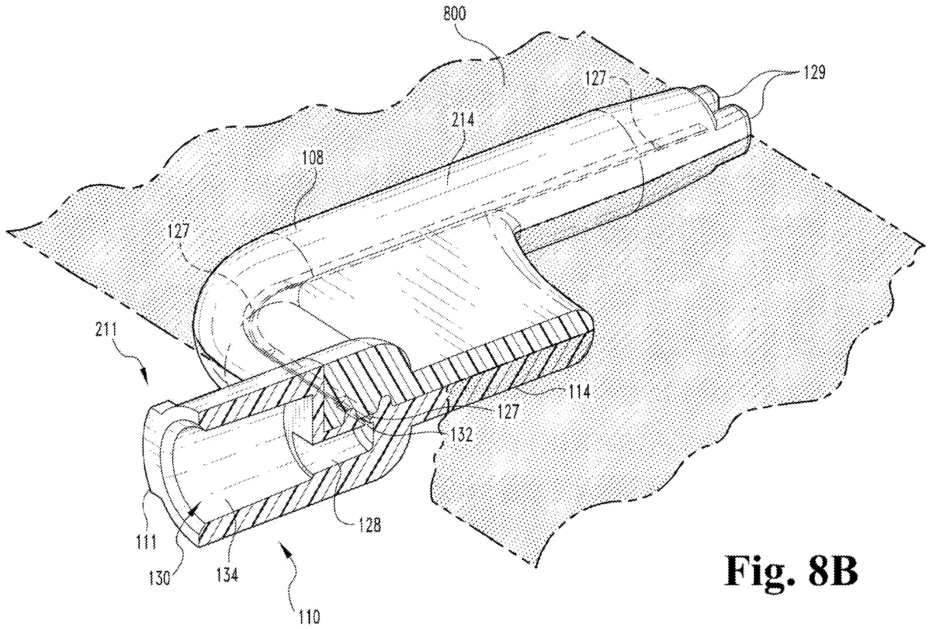

FIG. 8 is a cross-sectional view of an assembled applicator in accordance with some embodiments;
FIG. 8A is a magnified cross-sectional view of a portion of the assembled applicator of FIG. 8 and indicated by circle 8A; and
FIG. 8B is a cross-sectional view of the assembled applicator of FIG. 8 showing a plane passing through the joint.

The scope of the invention is defined by the claims. Any references in the description to methods of treatment refer to the compounds, pharmaceutical compositions and medicaments of the present invention for use in a method for treatment of the human (or animal) body by therapy (or for diagnosis). This invention provides methods of controlling pain in an equine comprising transdermally administering a composition according to the claims comprising fentanyl, a penetration enhancer, and a volatile liquid, wherein the composition is a solution. The invention also provides a single unit dose of the composition.

The present invention provides several advantages of fentanyl delivery to equines. First, the present invention achieves unexpected advantages in pharmacokinetic parameters such as absorption in equines compared to results observed in other species. Use of the present invention in equines results in absorption that is faster, more extensive, and at a higher rate compared to other species such as canines.

Second, when administered for the control of pain, the present invention allows for an unexpectedly lower therapeutically effective dose to be administered to equines compared to dosage amounts administered to other species. Specific advantages of the lower doses given to equines include a reduced possibility for abuse as well as a beneficial safety profile for individuals administering fentanyl to equines according to the claimed methods. Third, use of the present invention provides advantages compared to fentanyl administered to equines in a patch formulation. For example, the present invention provides for fentanyl administration in a formulation that is not easily damaged or consumed by equines compared to fentanyl administered to equines in a patch formulation. Moreover, because the present invention is a transdermal solution and not a device, the invention does not require the maintenance of skin contact in equines to maintain appropriate fentanyl absorption. Fourth, without a fentanyl reservoir, diversion and illicit use of the present invention, as well as disposal concerns outside the control of a licensed veterinarian, are minimized.

Fifth, the present invention achieves unexpected advantages in pharmacokinetic parameters in equines compared to results observed in patch formulations of fentanyl administered to equines. For example, the pharmacokinetic properties of fentanyl in equines have been described following transdermal administration of fentanyl via a transdermal patch formulation. The average time to achieve the maximum plasma concentration (Tmax) following transdermal administration of fentanyl (0.033 - 0.043 mg/kg) via a patch was approximately 8 hours. Furthermore, the maximum plasma concentration (Cmax) following transdermal administration of fentanyl via a patch was approximately 2.6 ng/ml.

In another example, transdermal administration of fentanyl via a patch formulation at a dose of 0.06-0.067 mg/kg resulted in extremely variable pharmacokinetic parameters of fentanyl in equines. The pharmacokinetic variability included a Tmax from 8.5-14.5 hours and a Cmax ranging from 0.67-5.12 ng/ml.

In comparison, the present invention requires a shorter duration of time to reach Tmax and achieves a much greater Cmax. Thus, the present invention allows equines to achieve greater plasma concentrations of fentanyl more quickly compared to fentanyl administered via a traditional patch application.

The methods according to the present invention utilize administration of a composition to an equine for control of pain. As used herein, the term "equine" refers to any member of the horse family, including for example horses, ponies, miniature horses, miniature ponies, donkeys, mules, and zebras.

As used herein, the terms "control of pain" or "controlling pain" refer to preventing, minimizing, or eliminating pain in an equine. As used herein, the term "pain" represents all categories of pain, including traumatic pain resulting from tissue injury, post-surgical pain, burn pain, inflammatory pain, pain associated with disease (such as cancer, arthritis, or infection), pain associated with nerve damage, neuropathy, and other forms of neuralgic, neuropathic and idiopathic pain syndromes, and specific organ or tissue pain, such as ocular and corneal pain, bone pain, heart pain, skin pain, visceral (kidney, gastrointestinal, colic, etc.) pain, joint pain, dental pain, soft tissue pain, and muscle pain. The term "pain" also includes pain of varying severity, *i.e.* mild, moderate and severe pain, as well as acute and chronic pain.

In some embodiments of the present invention, the methods utilize administration of a composition to an equine for control of pain during an effective period of time. As used herein, the term "effective period of time" comprises a period of at least 6 hours. In some embodiments, an effective period of time comprises a period of at least 6 hours, a period of at least 8 hours, a period of at least 12 hours, a period of at least 24 hours, a period of at least 48 hours, a period of at least 72 hours, or a period of at least 96 hours.

The composition administered according to the present invention comprises fentanyl, a penetration enhancer, and a volatile liquid. Fentanyl is a full µ-opioid receptor agonist and is also known by chemical names such as *N*-Phenyl-*N*-[1-(2-phenylethyl)-4-piperidinyl]propanamide, *N*-(1-phenethyl-4-piperidyl)-propionanilide, or *N*-(1-phenethyl-4-piperidinyl)-*N*-phenylpropionamide. The chemical structure of fentanyl is:

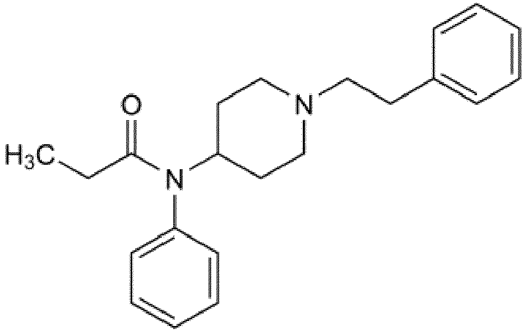

As used herein, the term "fentanyl" refers to fentanyl base, and pharmaceutically acceptable salts of fentanyl. The term "pharmaceutically acceptable salt" refers to an addition salt that exists in conjunction with the acidic or basic portion of fentanyl. Such salts include the pharmaceutically acceptable salts listed in HANDBOOK OF PHARMACEUTICAL SALTS: PROPERTIES, SELECTION AND USE, P. H. Stahl and C. G. Wermuth (Eds.), Wiley-VCH, New York, 2002, which are known to the skilled artisan. Pharmaceutically acceptable salts of an acid addition nature are formed when fentanyl and any of its intermediates containing a basic functionality are reacted with a pharmaceutically acceptable acid. Pharmaceutically acceptable acids commonly employed to form such acid addition salts include inorganic and organic acids. Pharmaceutically acceptable salts of a base addition nature are formed when fentanyl and any of its intermediates containing an acidic functionality are reacted with a pharmaceutically acceptable base. Pharmaceutically acceptable bases commonly employed to form base addition salts include organic and inorganic bases.

In addition to pharmaceutically acceptable salts, other salts are included in the present invention. They may serve as intermediates in the purification of compounds or in the preparation of other pharmaceutically-acceptable salts, or are useful for identification, characterization or purification.

As used herein, the term "penetration enhancer" refers to a chemical that improves the transport of drugs across the skin barrier. Penetration enhancers may act by disrupting the packing of skin lipids and thus altering the barrier nature of the stratum corneum, by changing the partitioning behavior of the drug at the stratum corneumviable epidermis interface, or by affecting the thermodynamic activity of the drug.

The penetration enhancers can be of low toxicity to the skin and are typically promoters of percutaneous absorption. In some embodiments, the penetration enhancer is a lipophilic chemical. Penetration enhancers and uses thereof are described, for example, in U.S. Patent Nos. 6,299,900, 6,818,226, and 6,916,486.

The penetration enhancers according to the present invention are particularly suitable for transdermal delivery of analgesics through the skin of an animal such as an equine. A number of penetration enhancers are known in the art. Penetration enhancers include fatty acids, fatty acid esters, fatty alcohols, glycols and glycol esters, 1,3-dioxolanes and 1,3-dioxanes, macrocyclic ketones containing at least 12 carbon atoms, oxazolidinones and oxazolidinone derivatives, alkyl-2-(N,N-disubstituted amino)-alkanoate esters, (N,N-disubstituted amino)-alkanol alkanoates, sunscreen esters, and mixtures thereof. In some embodiments, penetration enhancers are selected from the group consisting of oleic acid, oleyl alcohol, cyclopentadecanone (CPE-218™), sorbitan monooleate, glycerol monooleate, propylene glycol monolaurate, polyethylene glycol monolaurate, 2-n-nonyl 1,3-dioxolane (SEPA™), dodecyl 2-(N,N-dimethylamino)-propionate (DDAIP) or its salt derivatives, 2-ethylhexyl 2-ethylhexanoate, isopropyl myristate, dimethyl isosorbide, 4-decyloxazolidinon-2-one (SR-38™, TCPI, Inc.), 3-methyl-4-decyloxazolidinon-2-one, octyl dimethyl-para-aminobenzoate, octyl para-methoxycinnamate, octisalate, and mixtures thereof. The penetration enhancer of claim 1 is octyl salicylate.

In some embodiments, penetration enhancers can be sunscreen esters such as the compounds described in U.S. Patent Serial Number 6,299,900. For example, the compounds can be safe skin-tolerant ester sunscreens of formula: 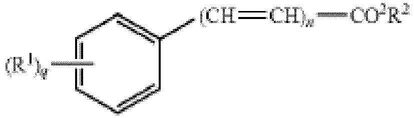
wherein R¹ is hydrogen, lower alkyl, lower alkoxy, halide, hydroxy or NR³R⁴;
R.sup.² is long chain alkyl;
R³ and R⁴ are each independently hydrogen, lower alkyl or R³ and R⁴ together with the nitrogen atom to which they are attached form a 5- or 6-membered heterocyclic ring;
n is 0 or 1; and
q is 1 or 2.

In some embodiments, penetration enhancers are esters having a long chain alkyl para-aminobenzoate, long chain alkyl dimethyl-para-aminobenzoate, long chain alkyl cinnamate, long chain alkyl methoxycinnamate or long chain alkyl salicylate. In some embodiments, penetration enhancers are selected from the group consisting of octyl dimethyl-para-aminobenzoate ("Padimate O"), octyl para-methoxycinnamate, octyl salicylate (also known as octisalate) or mixtures thereof. The present compositions comprise the penetration enhancer octyl salicylate.

As used herein, the term "volatile liquid" refers to any pharmacologically suitable liquid composition known in the art. For example, a volatile liquid may be readily vaporizable at low temperatures or tends to evaporate rapidly. Once applied to the skin, rapid evaporation of volatile liquids according to the present invention can result in super-saturation of other ingredients of the composition. In some embodiments, volatile liquids according to the present invention include safe skin-tolerant solvents. In some embodiments, the volatile liquid is a lower alkyl alcohol or a mixture of such alcohols. In some embodiments, the volatile liquid is selected from the group consisting of ethanol, ethyl acetate, isopropanol, acetone, ethyl formate, methyl acetate, methyl ethyl ketone, pentane and chloroform, or mixture thereof. In other embodiments, the volatile liquid is ethanol or isopropanol or mixtures thereof. In one embodiment, the volatile liquid is isopropanol.

The uses according to the present invention utilize administration of a composition wherein the composition is in a solution. In some embodiments of the present invention, the composition comprises fentanyl, a penetration enhancer, and a volatile liquid at various amounts based on a weight per volume of the solution. In some embodiments, the composition comprises on a weight basis from about 0.1 to about 10% of fentanyl, from about 0.1 to about 10% of the penetration enhancer, and from about 80% to about 99.8% of the volatile liquid. In another embodiment, the composition comprises on a weight basis from about 1 to about 10% of fentanyl, from about 1 to about 10% of the penetration enhancer, and from about 80% to about 98% of the volatile liquid. In another embodiment, the composition comprises on a weight basis from about 2 to about 8% of fentanyl, from about 2 to about 8% of the penetration enhancer, and from about 84% to about 96% of the volatile liquid. In another embodiment, the composition comprises on a weight basis from about 3 to about 7% of fentanyl, from about 3 to about 7% of the penetration enhancer, and from about 86% to about 94% of the volatile liquid. In yet another embodiment, the composition comprises on a weight basis from about 1 to about 5% of fentanyl, from about 1 to about 5% of the penetration enhancer, and from about 90% to about 98% of the volatile liquid. In another embodiment, the composition comprises on a weight basis about 5% of fentanyl, about 5% of the penetration enhancer, and about 90% of the volatile liquid.

Furthermore, the uses according to the present invention utilize administration of a composition wherein the administration is a transdermal administration. As used herein, the term "transdermal" has its ordinary meaning in the art and refers to passage of an agent across at least one skin layer of an animal, for example an equine. Further, the term "transdermal" is used co-terminously with the term "topical" in describing the application of agents to the skin. Both terms "topical" and "transdermal" are used herein in the broadest sense to refer to administration of a drug to the skin surface of an animal so that the drug passes through the skin layer. Unless otherwise stated or implied, the terms topical drug delivery and transdermal drug delivery are used interchangeably. From a strict drug-delivery perspective, "transdermal" is sometimes used to refer only to systemic delivery through the skin whereas "topical" requires only delivery into or on the skin for local effect. The invention described in this specification is equally applicable to both transdermal and topical modes of delivery, and is described here as "transdermal" only for convenience. The uses according to the present invention may utilize transdermal administration of the composition wherein the composition is desirably not a transdermal patch.

In carrying out the uses of this invention, the amount of fentanyl in the composition is adequate to achieve a therapeutic effect. As used herein, the term "therapeutically effective amount" refers to an amount which gives the desired benefit to an equine and includes both treatment and prophylactic administration. The amount may vary from one equine to another and can depend upon a number of factors, including the overall physical condition of the equine and the underlying cause of the condition to be treated.

The amount of fentanyl used for the controlling pain gives an acceptable rate of change and maintains desired response at a beneficial level. A therapeutically effective amount of the composition used in the methods of this invention may be readily ascertained by one of ordinary skill in the art using publicly available materials and procedures. In one embodiment of the present invention, the therapeutically effective amount of fentanyl to be delivered can be quantified by determining milligrams of fentanyl per kilogram of equine body weight. According to the claims, the therapeutically effective amount of fentanyl in the composition is between 0.01 and 0.1 milligrams per kilogram of equine body weight. In some embodiments, the therapeutically effective amount of fentanyl in the composition can be present in an amount of between about 0.01 and about 0.08 milligrams per kilogram of equine body weight. In some embodiments, the therapeutically effective amount of fentanyl in the composition can be present in an amount of between about 0.02 and about 0.06 milligrams per kilogram of equine body weight. In one embodiment, the therapeutically effective amount of fentanyl present in the composition is about 0.05 mg/kg. In one embodiment, the therapeutically effective amount of fentanyl present in the composition is about 0.04 mg/kg. In one embodiment, the therapeutically effective amount of fentanyl present in the composition is about 0.03 mg/kg. In one embodiment, the therapeutically effective amount of fentanyl present in the composition is about 0.02 mg/kg. In one embodiment, the therapeutically effective amount of fentanyl present in the composition is about 0.01 mg/kg.

In one embodiment, the composition according to the present invention comprises on a weight basis about 5% of fentanyl in a solution (*i.e.,* 50 mg/mL) and the therapeutically effective amount of fentanyl present in the composition is about 0.04 mg/kg. In another embodiment, the composition according to the present invention comprises on a weight basis about 2.5% of fentanyl in a solution (*i.e.,* 25 mg/mL) and the therapeutically effective amount of fentanyl present in the composition is about 0.04 mg/kg. In yet another embodiment, the composition according to the present invention comprises on a weight basis about 2.3% of fentanyl in a solution (*i.e.,* 23 mg/mL) and the therapeutically effective amount of fentanyl present in the composition is about 0.04 mg/kg.

In some embodiments, the therapeutically effective amount is an amount sufficient to achieve a minimum effective plasma concentration (MEC). Generally, MEC has been defined as the minimum plasma concentration of an analgesic that is sufficient to prevent a patient from requesting a supplementary analgesic. The MEC of fentanyl in humans has been established in a population of adults undergoing abdominal surgery. Following surgery, fentanyl was delivered at a basal IV infusion rate of 20 µg/hr with 20 microgram on demand boluses self administered by the patient when pain became unacceptable. A blood sample collected just prior to the patient administering additional analgesia was considered the MEC. Over 48 hours, the MEC ranged from 0.23 to 1.18 ng/mL (mean 0.63 ng/mL) and remained relatively constant within individual patients over the 48-hour study period. Thus, in humans where pain was alleviated at 0.2 ng/mL this remained constant over time as well as for those where pain was alleviated with 1.18 ng/mL. This suggests a 6-fold range of minimally effective fentanyl concentrations dependent on individual responsiveness.

Equines cannot request their own supplementary analgesia, thus quantifying the true MEC remains difficult and depends on an observer making inferences from presumed pain related behaviors displayed by equines. Despite these limitations, behavior-based studies could evaluate analgesia and plasma fentanyl concentration in equines to approximate analgesia and drug concentrations.

In some embodiments of the present invention, pain is associated with a surgery performed or to be performed on the equine. In one embodiment, the surgery performed or to be performed on the equine is an orthopedic surgery. As used herein, the term "orthopedic surgery" refers to a surgical procedure pertaining to the preservation or restoration of the function of the musculoskeletal system, its articulations, and associated structures.

In another embodiment, the surgery performed or to be performed on the equine is a soft tissue surgery. As used herein, the term "soft tissue surgery" refers to a surgical procedure pertaining to the preservation or restoration of the function of muscle, fat, fibrous tissue, blood vessels, or other supporting tissue of the body, for example tendons, ligaments, fascia, skin, nerves, or synovial membranes.

In some embodiments of the present invention, pain is associated with colic in the equine. As used herein, the term "colic" encompasses all forms of gastrointestinal conditions which cause pain as well as other causes of abdominal pain not involving the gastrointestinal tract. Colic may include a visceral component. In equines, colic can be treated by surgical methods and by non-surgical methods.

In some embodiments of the present invention, pain is associated with laminitis in the equine. As used herein, the term "laminitis" is associated with a soft tissue disorder in equines. In equines, laminitis can be treated by surgical methods and by non-surgical methods. Laminitis may be further categorized as acute laminitis or chronic laminitis.

In one embodiment of the present invention, the composition is contained in a multiple-dose vial prior to administration. The multiple-dose vial containing the composition of the present invention can be made of glass, plastic, or other material. In one embodiment of the present invention, the composition is administered as a multiple dose regimen.

In one embodiment of the present invention, the composition is administered as a single dose. In yet another embodiment of the present invention, the composition is administered as a single unit dose. As used herein, the term "unit dose" is a discrete amount of the composition comprising a predetermined amount of fentanyl. The amount of fentanyl is generally equal to the dosage of fentanyl which would be administered to an equine or a convenient fraction of such a dosage such as, for example, one-half or one-third of such a dosage.

According to the present invention, the terms "single dose" and "single unit dose" include embodiments wherein the composition can be transdermally administered as a single application and administered as multiple applications. In one embodiment, a single dose or single unit dose of the composition can be transdermally administered to an equine in a single application at one location on the equine's skin. In another embodiment, a single dose or single unit dose of the composition is transdermally administered to an equine in a single application at one location on the equine's skin, wherein the single application is about 0.9 mL of a solution of the composition. In one embodiment, a single dose or single unit dose of the composition can be transdermally administered to an equine in multiple applications at a single location on the equine's skin. In another embodiment, a single dose or single unit dose of the composition is transdermally administered to an equine in multiple applications at a single location on the equine's skin, wherein each application has up to about 0.9 mL of a solution of the composition. In one embodiment, a single dose or single unit dose of the composition can be transdermally administered to an equine in multiple applications at more than one location on the equine's skin. In another embodiment, a single dose or single unit dose of the composition is transdermally administered to an equine in multiple applications at more than one location on the equine's skin, wherein each application has up to about 0.9 mL of a solution of the composition. In embodiments wherein multiple applications of the composition are utilized, the multiple applications can be administered to the equine over a reasonable duration of time.

In one embodiment, the composition can be transdermally administered to the equine at a location from which the hair is clipped. In another embodiment, the composition can be transdermally administered to the equine at a location from which the hair is not clipped. In one embodiment, the composition can be transdermally administered to the equine at a location at which the skin is cleaned. For example, the skin of the equine can be cleaned with a disinfectant solution. In another embodiment, the composition can be transdermally administered to the equine at a location at which the skin is not cleaned.

In one embodiment of the present invention, the composition is transdermally administered to an equine at a dorsal location of the equine. According to the methods of the present invention, the term "dorsal" has its ordinary meaning and as used herein refers to the location on the top of the animal, *i.e.,* along the equine's back. In one embodiment of the present invention, the composition is transdermally administered to an equine at a ventral location of the equine. According to the methods of the present invention, the term "ventral" has its ordinary meaning and as used herein refers to the direction towards the abdomen of an equine, *i.e.,* along the underside of the equine's body.

In another embodiment of the present invention, the composition is transdermally administered to an equine at a location on the foreleg of the equine. According to the methods of the present invention, the term "foreleg" has its ordinary meaning and as used herein refers to any proximal or distal location on either of an equine's forelegs. In yet another embodiment of the present invention, the composition is transdermally administered to an equine at a location on the hindleg of the equine. According to the methods of the present invention, the term "hindleg" has its ordinary meaning and as used herein refers to any proximal or distal location on either of an equine's hindlegs.

In one embodiment of the present invention, the composition is transdermally administered to an equine at a location on the neck of the equine. According to the methods of the present invention, the term "neck" has its ordinary meaning and as used herein refers to any location on either side of the neck. In one embodiment of the present invention, the composition is transdermally administered to an equine at a location on the dorsum of the equine. According to the methods of the present invention, the term "dorsum" has its ordinary meaning and as used herein refers to any location on the body of the equine from the neck to the end of the spine. In another embodiment of the present invention, the composition is transdermally administered to an equine at a location near the mane.

In yet another embodiment of the present invention, the composition is transdermally administered to an equine at a location underneath the mane. This embodiment may offer further advantages of the present invention because of the mane may protect the administrator (e.g., a veterinarian) from the site of administration. Thus, accidental absorption of the composition by the administrator may be minimized.

The compositions of the present invention include those that also optionally contain one or more other active ingredients, in addition to fentanyl. As used herein, the term "active ingredient" or "therapeutic ingredient" refers to a therapeutically active compound, as well as any prodrugs thereof and pharmaceutically acceptable salts, hydrates, and solvates of the compound and the prodrugs. Other active ingredients may be combined with fentanyl and may be either administered separately or in the same pharmaceutical formulation. The amount of other active ingredients to be given may be readily determined by one skilled in the art based upon therapy with fentanyl.

In one embodiment of the methods according to the present invention, a transdermal dispensing apparatus can be used to administer the composition to an equine. In one embodiment, the transdermal dispensing apparatus can be an applicator for dispensing a formulation herein described to an animal. An example of a transdermal dispensing apparatus is described, for example, in PCT Patent Application No. PCT/US2010/053206 and in U.S. Patent Application No. 12/581,658.

One example of an applicator comprises a housing including first and second sections coupled together, the first and second sections defining a channel therebetween that includes at least one outlet; a hub integral with the first section and extending therefrom, the hub defining a conduit; and a bent path connecting the conduit to the channel. In accordance with this embodiment, the conduit, the bent path and the channel are fluidly connected.

In accordance with still another aspect of the present invention, a method is provided for dispensing the formulation herein described from an applicator of the type having a housing including first and second sections coupled together to form a channel and a hub extending from the housing, the hub being attachable to a syringe. The method comprises attaching the hub to a syringe containing a formulation; placing an outlet of the applicator on or near the animal; causing the formulation to be released from the syringe into the applicator; passing the formulation through the hub, through a bent path and then into the channel; and dispensing the formulation from the applicator through the outlet.

The applicators described herein can be particularly useful for transdermally delivering doses of controlled veterinary substances (*e.g*., fentanyl) to the coat and skin of an animal, which may include an equine. In certain exemplary embodiments, the drug delivery device includes an applicator device or tip that is compatible with a standard luer lock syringe and consists of a housing that allows the formulation to be spread over a surface area of the animal's skin or coat. To accomplish this, the applicator body includes one or more outlets that are in the form of legs or tines configured to penetrate the haircoat of the animal and thereby deliver the drug directly to the animal's skin or coat. In certain aspects, the outlet(s) further includes a pair of spaced prongs or feet that extend from its distal end, thereby allowing the formulation to be freely dispensed onto the surface of the animal. More particularly, because the spaced feet extend outwardly from the distal end of applicator, they are the only structural portion of the assembled applicator that directly contact and seal against the surface of the animal. Moreover, since the outlet opening is positioned between the spaced apart prongs and in such a manner that it does not directly contact or seal against the surface of the animal during a dispensing operation, the formulation is able to be freely dispensed and spread onto the animal without being physically impeded or interrupted.

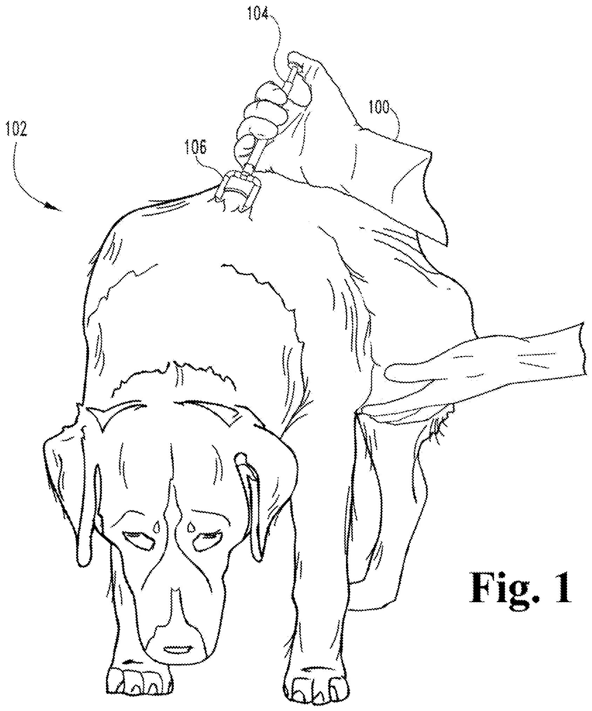

A non-limiting illustration of an assembled applicator coupled to a fluid delivery device in accordance with the present teachings is shown in FIG. 1. More specifically, FIG. 1 depicts a perspective view of a user 100 dispensing a formulation herein described onto an animal 102. In accordance with this exemplary and non-limiting illustration, a fluid delivery device 104 containing the formulation is releasably attached to an applicator device 106 and then placed on or near the surface of the animal 102. While this illustrative embodiment shows the fluid delivery device 104 as a standard syringe, it should be understood and appreciated herein that delivery of the formulation may be accomplished by any known fluid delivery device or connector that is releasably attachable to the applicator device 106 and having a reservoir for holding and/or storing the formulation to be dosed or dispensed. Other such non-limiting and illustrative fluid delivery devices that may also be used include, but are not limited to, syringes, catheters, hubbed needles, IV tubes and cylinder fluid delivery devices.

As will be explained in detail below, the applicator devices 106 generally consist of at least two parts or halves (*i.e.,* sections 114 and 214) that are coupled or assembled together to form the applicator structure. Unlike many other traditional applicator devices that consist of either one applicator part or two structurally complementary parts, the devices include two sections 114, 214 that are somewhat complementary in terms of structure, yet specifically shaped in such a manner that once assembled, the formulation can be dispensed therefrom without experiencing much associated leakage or residual buildup. More particularly, the sections 114, 214 are structurally shaped such that when they are coupled together, the formulation is discouraged from leaking out of the applicator body. In addition, the structural orientation of the dispensing passageway that is created between the first and second sections is shaped in such a manner that substantially all of the formulation is encouraged from being dispensed from the applicator device during a dispensing operation. As such, it should be understood and appreciated herein that at least some of the unexpected advantages are influenced by the resultant shape and configuration of the dispensing passageway that is formed by the assembled applicator sections.

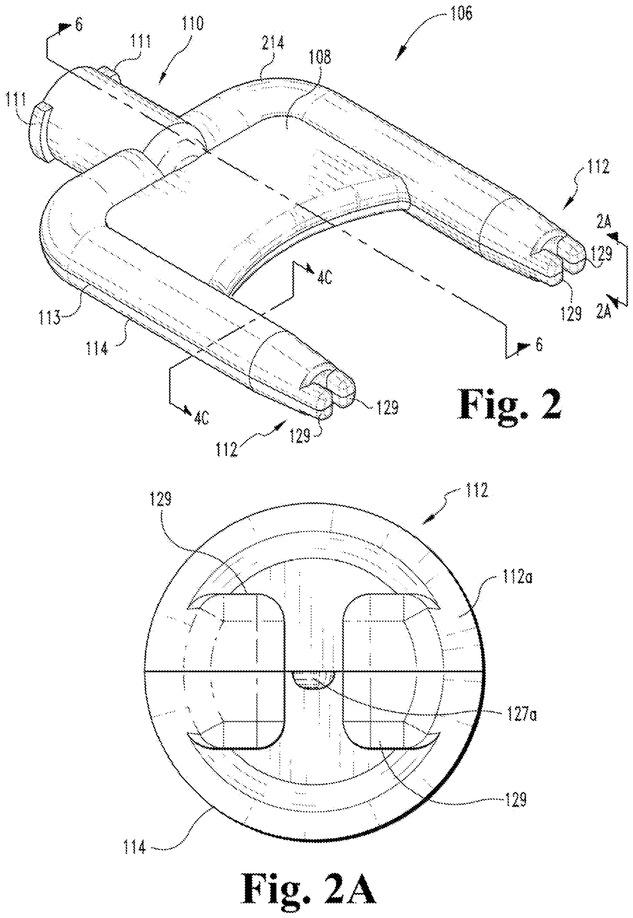

Moving now to FIG. 2 a perspective view of a fully assembled applicator 106 is shown. The applicator 106 includes a housing or body 108 defining an inlet hub 110 and an outlet 112. As will be explained in more detail below, the inlet hub 110 is attachable to the drug delivery device 104 during a dispensing operation, whereas the outlet portion 112 is capable of penetrating the haircoat of an animal so that the formulation can be appropriately dispensed therefrom and onto the surface of the animal. The applicator 106 can be made from polyethylene, polypropylene, polyvinyl acetate, polystyrene, polyethylene terephthalate, polybutylene terephthalate, and polytetrafluoroethylene, and the like.

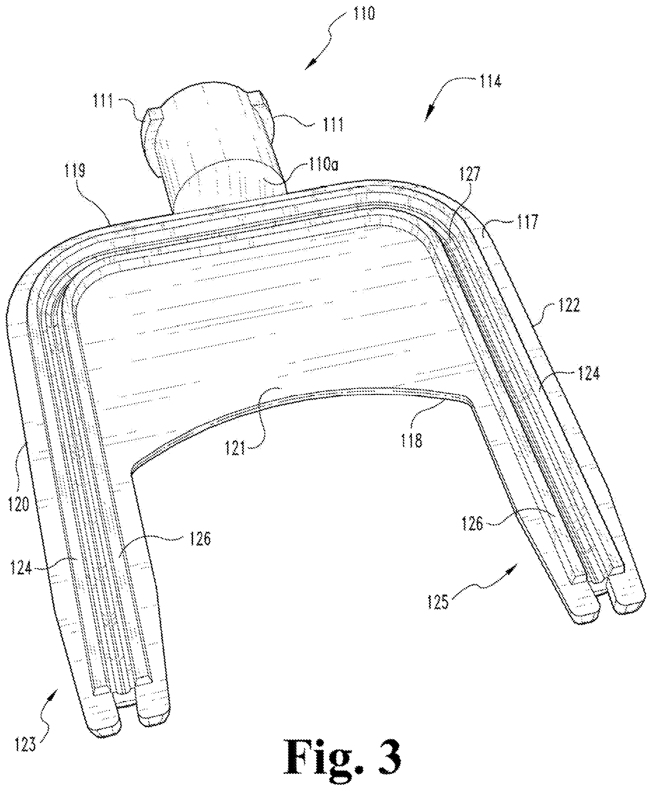

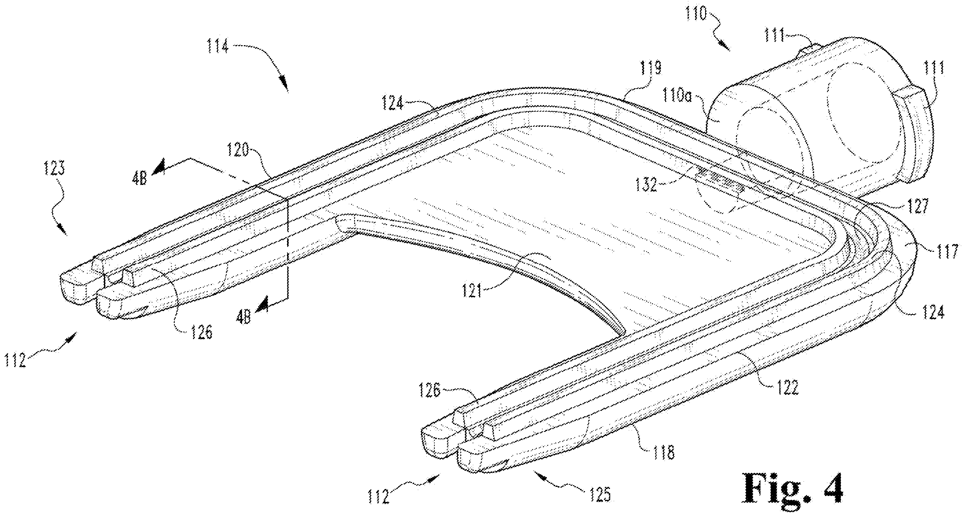

In terms of assembly, the applicator 106 comprises first and second sections or faces (114, 214) that are coupled or assembled together to form the housing 108. As shown in FIGS. 3 and 4, the first section 114 includes a top surface 117, a bottom surface 118, a back edge 119 integral with the inlet hub 110 and first and second sides 120, 122, the first and second sides being defined by a pair of substantially parallel outlet ends or legs 123, 125 that extend from and partially surround a substantially flat middle section 121 that is disposed between the first and second sides 120, 122. Extending upwardly from the top surface 117 of the first preassembled section 114 and positioned substantially along its outer periphery are a pair of ribs 124, 126 that are spaced from each other in a parallel fashion. In certain exemplary embodiments, the ribs 124, 126 are trapezoidal shaped and have four sides with the top and bottom sides being parallel to one another. In accordance with this exemplary embodiment, the spaced ribs 124, 126 have a groove or channel 127 that is formed therebetween.

In certain aspects, the groove 127 is sunken or depressed below the top surface 117 of the first section, thereby creating a channel for delivering the formulation to the outlet ends 123, 125 and ultimately onto the animal. To achieve the sunken channel formation, the groove 127 is provided as a depression below the surface 117 and has a substantially semi-circular shape. A more detailed and non-limiting exemplary illustration of this semi-circular geometry can be seen with reference to FIG. 4B, which illustrates a cross-sectional view of the first section 114 taken along line 4B of FIG. 4. While this exemplary illustration shows the groove or channel 127 being semi-circular in shape, it should be understood and appreciated herein that any known geometric shape useful for establishing a channel that permits a fluid or other such liquid agent to travel therethrough is envisioned and can be used.

As explained above, it should be understood and appreciated herein that the first preassembled section 114 is configured to be coupled to and molded with the second preassembled section 214 to form a fully assembled applicator device 106. In addition, the channel or groove 127 that is formed between the ribs 124 and 126 is positioned and shaped in such a manner that a fluid passageway or conduit for dispensing the formulation is formed between the fluid delivery device 104 and the dispensing end of the outlet 112 once section 114 is coupled to and molded with section 214.

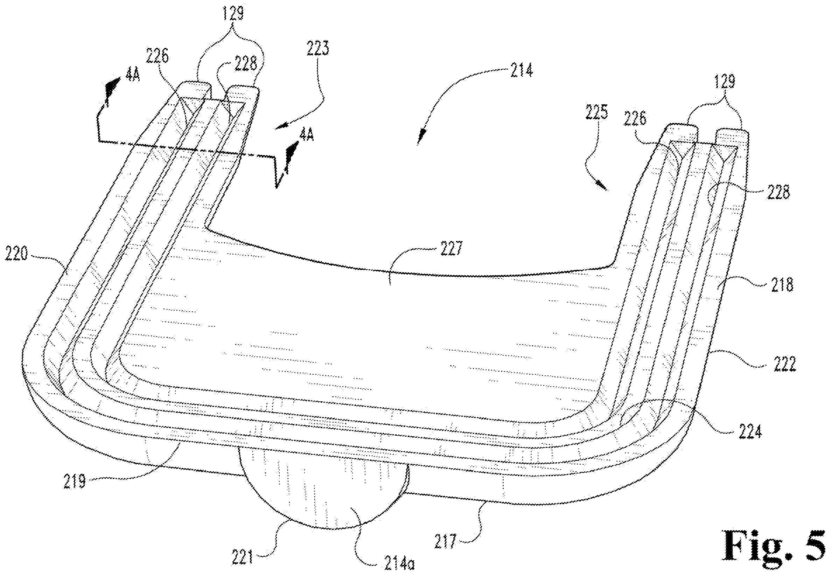

Moving now to FIG. 5, the second preassembled section 214 has a shape that is substantially similar to and which complements the first preassembled section 114; however, it does not have a corresponding inlet hub portion or a rib and groove arrangement like that of the first section 114. Instead, the second section 214 includes a top surface 217, a bottom surface 218, and a back edge 219 having a rounded portion 221 that is substantially centrally located along the back edge 219 and is configured to substantially align with the inlet hub 110 portion of the first section during assembly. To achieve this alignment, the inlet hub 110 has a flat end portion 110a that is complementarily shaped to and configured to seamlessly mate with a flat end portion 214a of the second section 214. The second preassembled section 214 also includes first and second sides 220, 222 that are defined by a pair of substantially parallel outlet ends or legs 223, 225 that extend from and partially surround a substantially flat middle section 227 that is disposed between the first and second sides 220, 222. Extending outwardly from the bottom surface 218 of the second section and positioned substantially along its outer periphery is a ledge or energy director 224 that is formed by a pair of spaced grooves 226, 228. A more detailed and non-limiting exemplary illustration of this geometric configuration can be seen with reference to FIG. 4A, which illustrates a cross-sectional view of the second section 214 taken along line 4A of FIG. 5.

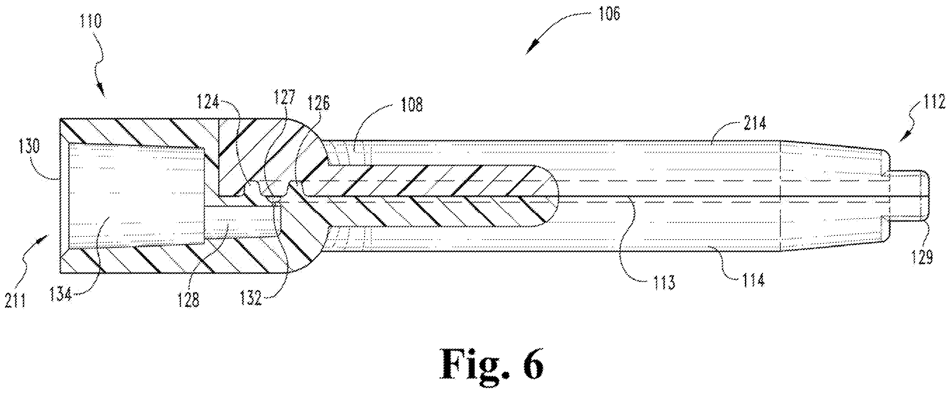

During assembly of the applicator 106, the pair of spaced ribs 124, 126 of the first preassembled section 114 are configured to substantially align with (and mate) the spaced grooves 226, 228 of the second section 214, thereby forming the passageway or channel 127 for dispensing the formulation. In accordance with certain exemplary embodiments, the passageway 127 is asymmetric relative to a seamless joint 113 that attaches the first and second sections 114, 214 together. A fully assembled view of the first and second sections 114, 214 aligned and mated together can be seen in FIGS. 4C and 6, which respectively depict a cross-sectional view of the assembled applicator 106 from FIG. 2 taken along line 4C and a cross-sectional side view of the assembled applicator 106 from FIG. 2 taken along line 6.

As can be seen particularly in FIG. 4C, after the first and second sections are welded together, the spaced ribs 124, 126 meld into grooves 226, 228 so that a seamless joint 113 is formed between the two faces 114, 214, and the channel 127 is formed therebetween. In particular, a substantially flat portion of the channel 127 is defined by the seamless joint 113. Once fully assembled, the channel 127 creates a fluid passageway between the inlet hub 110 and the one or more outlets 112. As shown in FIG. 2A, the distal end 112a of the applicator's outlet is open (see reference numeral 127a) so the formulation can be emptied from channel 127 during a dispensing application.

In another embodiment, the first and second preassembled sections 114, 214 can be coupled together to form an assembled applicator 106 by various known plastic molding and manufacturing methods. However, in certain aspects, the applicator 106 is formed by ultrasonically welding the first and second preassembled sections 114, 214 together. In accordance with this exemplary and non-limiting embodiment, the first and second preassembled sections 114, 214 are mated and aligned together as explained above, and an ultrasonic weld, for instance along the ledge 224, is initiated to thereby cause the sections to seamlessly meld or join together. As is readily known and appreciated by those of skill in the plastics manufacturing and welding arts, the process of ultrasonically welding two plastic parts together along an energy director that has been formed into one of the preassembled parts allows a bond to be formed that is tensile and resists the tendency of forces to tear the bond apart. Specifically, the ultrasonic energy melts the point contact between the parts, thereby creating a seamless joint. Moreover, these types of welds can typically be strengthened by either increasing the weld depth, or increasing the size of the energy director to provide a larger weld area. Accordingly, it should be understood and appreciated herein that the precise shapes and sizes of the preassembled components described herein are not essential, particularly as a skilled artisan would understand how to maximize the size and shapes of the components to achieve the best welded result for the specific dispensing applicator device to be assembled.

There are, however, advantages to the embodiment of the applicator 106 illustrated in FIGS. 4A, 4B, and 4C. In particular, the structure of the first section 114 and second section 214 is advantageous in forming a substantially semi-circular channel 127 that encourages a formulation to be dispensed therethrough while leaving only a minimal amount of residual remaining in the channel after use. One reason for this is because the weld path, *i.e.,* seamless joint 113, is disposed close to the fluid path, *i.e.,* channel 127. Another reason is because the channel 127 has a substantially flat portion, the ribs 124, 126 can be positioned closer to one another. As such, the channel 127 can be smaller thereby reducing the overall volume of the channel, which effectively reduces the amount of residual formulation remaining in the channel after dispensing the agent therethrough.

Another advantage with the illustrated embodiment of the applicator 106 is the shape of the grooves 226, 228 and the ledge 224 in the second section 214. Each groove is substantially V-shaped and the ledge 224 is substantially flat, as shown in FIG. 4A, such that when the first and second preassembled sections 114, 214 are mated and aligned together there is very little, if any, flash remaining in the channel 127. During ultrasonic welding, for example, the ultrasonic energy melts the energy director, *i.e.,* ledge 224, to form the joint 113 between the first and second sections 114, 214. In FIG. 4C, after the first section 114 and second section 214 are welded together, the channel 127 is formed without flash forming in the channel. Flash can disrupt or obstruct the flow of the formulation passing through the channel 127. Larger amounts of residual fluid can remain in the channel after the formulation is dispensed when flash is present in the channel 127. By reducing or eliminating flash, the channel 127 maintains a substantially semi-circular shape therethrough, which as described above reduces the amount of residual formulation remaining in the channel after use.

This is not, however, the case with differently shaped grooves and/or ledge in the second section. In FIG. 5A, for example, a different embodiment of a second section 514 having a top surface 517 and bottom surface 518 is shown. In addition, a different cross-section of the second section 514 is illustrated in which grooves 526, 528 are trapezoidal. The trapezoidal grooves 526, 528 are complementary to the trapezoidal ribs 124, 126 of the first section 114 (FIG. 5B). An energy director or ledge 524 of the second section 514 is substantially flat and therefore similar to the ledge 224 in FIG. 4A. As can be seen in FIG. 5C, after the first and second sections are welded together, the spaced ribs 124, 126 meld into grooves 526, 528 so that a seamless joint 113 is formed between the two faces 117, 518, and the channel 127 is formed therebetween. Unlike the semi-circular channel 127 shown in FIG. 4C, however, the mating of the trapezoidal grooves 526, 528 with the trapezoidal ribs 126, 128 produces flash 540 which fills a portion of the channel 127. The flash 540 reduces the size of the channel 127 such that the channel 127 no longer is semi-circular. One reason flash is produced in the channel is due to the difficulty of welding the trapezoidal grooves 526, 528 and the trapezoidal ribs 126, 128.

In FIG. 7A, another embodiment of a second section 714 having a top surface 717 and bottom surface 718 is shown. Moreover, the second section 714 includes grooves 726, 728 which are V-shaped and therefore similar to the grooves 226, 228 of FIG. 4A. The second section 714, however, also includes an energy director or ledge 724 that is not flat. Instead, the ledge 724 is pressed above the bottom surface 718 and has a semi-circular cross-section. The shape of the ledge 724 complementarily corresponds with the semi-circular channel 127 of the first section 114 shown in FIG. 7B. As can be seen in FIG. 7C, as the first and second sections are welded together, the spaced ribs 124, 126 meld into grooves 726, 728 so that a seamless joint 113 is formed between the two faces 117, 718, and the channel is formed therebetween. The channel 127 formed between the first and second sections has a substantially circular crosssection, but flash 740 forms in the channel thereby inhibiting flow therethrough. Flash is produced in the channel 127 due to the difficulty of welding the two sections together. As can be seen in FIG. 7A, for example, the ledge 724 is no longer substantially flat. In particular, there is very little material along the ledge 724 that contacts the first section 114 for ultrasonically welding the two sections together. Thus, to ensure a proper bond is formed to hold the first and second sections together, flash fills along the edges of the channel 127. Therefore, while it should be understood and appreciated herein that the precise shapes and sizes of the preassembled components described herein are not essential, it is advantageous for the preassembled components to comprise shapes and sizes that facilitate little to no flash.

A more detailed description of the various parts of the applicator 106 will now be provided. As is particularly shown in FIGS. 6, 8, 8A and 8B, the inlet hub 110 is fluidly connected to the first section 114 by way of a path 128 that is disposed between a pair of openings 130, 132. As should be understood and appreciated herein, the fluid connection between the inlet hub 110 and the first section 114 defines a conduit for receiving the formulation from the fluid delivery device 104 to the groove or channel 127. More particularly, the inlet hub 110 has a first opening 130 that is disposed at the proximal end 211 of the inlet hub 110 and functions as an insertion hole for receiving the dispensing end of the fluid delivery device (such as device 104 in FIG. 1). Opposite the first opening 130 is a second opening 132, which is fluidly connected to the groove or channel 127 of the housing 108. As such, the inlet hub 110 is designed to functionally form an opening for the fluid delivery device 104 so that the formulation can be easily and conveniently dispensed therefrom.

The inlet hub 110 has a pair of winged ears 111 adapted to lock to the fluid delivery device (not shown). More particularly, the fluid delivery device (e.g., device 104 in FIG. 1) is inserted into first opening 130 and securely attached to inlet hub 110 by any fastening means known in the art. Exemplary connection means include, but are not limited to, luer lock connections. Luer lock connections are well known in the field of medicine and are typically used for coupling a syringe or other such liquid or gas source to a catheter line or medical device. Moreover, as will be appreciated and understood by those skilled within the relevant art, the luer connectors may be female or male in orientation and may function as luer-locking devices, luer-slip connection devices or the like. In accordance with some specific aspects, the luer lock connection is achieved between the fluid delivery device 104 and the winged ears 111 of the inlet hub 110.

As can be appreciated from the discussion above, the flow path 128 undergoes a significant reduction in diameter along the direction of fluid flow (*i.e.,* from the inlet hub 110 to the distal end 112a of the outlet 112). This is necessary to adapt the applicator for connection to larger fluid delivery devices at the end of hub 110 on the one hand, and on the other hand to the very small channel 127 through which the fluid is moved before being dispensed from the outlet(s) 112. This reduction in diameter causes significant pressure within path 128, which in turn can cause leakage if there are any weak or vulnerable points such as weld seams along path 128. To address these structural issues, path 128 is bent or shaped such that it is circuitous in nature - *i.e.,* is not a direct route between the first and second openings 130, 132 and changes direction one or more times. In this manner, path 128 is formed entirely within a single section, section 114, of the applicator, which avoids weld seams being present for any of the structure that defines path 128. With reference to FIG. 8B, for example, the interface between the first and second sections, *i.e.,* joint 113 (FIG. 2), defines a plane 800 that passes therethrough. As shown in this illustrative embodiment, the path 128 is offset from the plane 800. By locating the flow path in one section of the applicator (as opposed to two sections defining a flow path therebetween) and consequently eliminating all weld seams within the area defined by the flow path 128, the occurrence of leakage as the fluid flows between the fluid delivery device 104 and the channel 127 is substantially reduced, if not eliminated.

The structure defining path 128 can be appreciated with reference to FIGS. 8 and 8A, wherein the conduit defined by the inlet hub 110 includes a short hollow cylindrical chamber 134 that is disposed between the first and second openings 130, 132 and terminates substantially centrally into the channel 127 at the second opening 132. Chamber 134 is typically designed such that it is dimensionally non-uniform (*i.e.,* varies in width and height between the first opening 130 and the second opening 132). According to this aspect, the internal diameter of the chamber 134 changes to achieve the reduction in diameter and configuration needed to maintain path 128 within a single section 114 of the applicator. As mentioned above, it has been found that this configuration avoids leakage of the formulation as it flows between the fluid delivery device and the channel.

In certain aspects, one or more tubes or other such enclosed tubular structures can be internally incorporated into the structural design of the present applicators. For instance, to avoid any associated leakage that may occur around the connection between the fluid delivery device and the applicator or along the joint 113 that is formed between the first and second molded sections 114, 214, one or more chambers can be internally added into the inlet hub 110 portion and/or within the formed channel 127 of the applicator body. While such additional structure can be incorporated into any of the embodiments without straying from the present teachings, it should be understood and appreciated herein that such structures are not required. More particularly, it has been found that utilizing the bent path orientation and complementary structural design of the applicator sections makes it possible to achieve a tubeless design that is not only free of manifolds, but is also capable of operating without resultant leakage.

In certain exemplary embodiments, the chamber 134 contains ridges, ledges, or other such similar structures to cause a bending configuration and stepped down diameter of the path 128. In still other aspects, the path 128 is positioned below the seamless joint 113 that is formed between the first and second sections 114, 214 and underneath the channel 127 formed therebetween.

In accordance with certain aspects, the second opening 132 directs the formulation into the channel in a direction that is substantially orthogonal to the lengthwise direction of the channel 127. Such exemplary embodiment can be seen, for instance, with reference to FIGS. 8 and 8A. While the dimensions and/or geometric shape of the second opening 132 can be adjusted to fit a specific drug delivery application, the opening 132 is substantially rectangular in shape.

In accordance with yet another illustrative aspect, the bent path 128 comprises a substantially semi-circular portion that is connected to the conduit for receiving the formulation from the fluid delivery device 104 and the channel 127. In accordance with this illustrative aspect, the bent path 128 terminates at the second opening 132, which in turn, is positioned substantially orthogonally relative to the substantially semi-circular portion of the bent path 128.

Once the formulation completely travels and circumnavigates the channel 127 and reaches the distal end 112a of the one or more outlets 112, it is now ready to be dispensed onto the surface or coat of the animal. As explained above, to spread the formulation evenly over a surface area of the animal, the outlet 112 can penetrate the animal's haircoat and thereby reach the animal's skin. To accomplish this, the outlet 112 may include one or more prongs 129 for assisting with the dispensing of the formulation onto the surface of the animal. In accordance with certain embodiments, the prongs 129 comprise spaced feet or tines that are configured to penetrate the haircoat of the animal 102 so that the applicator 106 can substantially reach or touch the surface of the animal's body during the dispensing of the formulation. This penetration allows a more efficient topical and transdermal release of the agent. In addition, those of skill in the drug delivery and fluid dispensing arts will understand and appreciate that the addition of prongs or other such structural projections from the outlet 112 will discourage capillary action or attraction (*i.e.,* will stop the formulation from moving upwardly along the outside of the outlet) from happening during the dispensing action. The minimization and/or elimination of such capillary action effects are particularly beneficial when dealing with formulations that can be considered harmful and/or dangerous.

The present invention also includes a single dose transdermal formulation comprising a therapeutically effective amount of a composition comprising fentanyl, a penetration enhancer selected from the group consisting of long chain alkyl para-aminobenzoate, long chain alkyl dimethyl-para-aminobenzoate, long chain alkyl cinnamate, long chain alkyl methoxycinnamate, long chain alkyl salicylate, octyl dimethyl-para-aminobenzoate, octyl para-methoxycinnamate, octyl salicylate, or mixtures thereof, and a volatile liquid selected from the group consisting of ethanol, ethyl acetate, isopropanol, acetone, ethyl formate, methanol, methyl acetate, methyl ethyl ketone, pentan, chloroform, or mixtures thereof suitable for administration. In some embodiments, the composition comprises fentanyl at a dose of about 0.01 and about 0.1 mg per kg of equine body weight. In some embodiments, the composition comprises fentanyl at a dose of about 0.04 mg per kg of equine body weight. In some embodiments, the single dose transdermal formulation controls pain in the equine for an effective period of time. In some embodiments, an effective period of time comprises a period of at least 6 hours, a period of at least 8 hours, a period of at least 12 hours, a period of at least 24 hours, a period of at least 48 hours, a period of at least 72 hours, or a period of at least 96 hours. In one embodiment, the single dose transdermal formulation is administered once every at least 6 hours. In one embodiment, the single dose transdermal formulation is administered once every at least 8 hours. In one embodiment, the single dose transdermal formulation is administered once every at least 12 hours. In one embodiment, the single dose transdermal formulation is administered once every at least 24 hours. In one embodiment, the single dose transdermal formulation is administered once every at least 48 hours. In another embodiment, the single dose transdermal formulation is administered once every at least 72 hours. In yet another embodiment, the single dose transdermal formulation is administered once every at least 96 hours.

According to the methods of the present invention, the term "single dose transdermal formulation" includes embodiments wherein the composition can be transdermally administered as a single application or as multiple applications. In one embodiment, the single dose transdermal formulation of the composition can be transdermally administered to an equine in a single application at one location on the equine's skin. In another embodiment, the single dose transdermal formulation of the composition can be transdermally administered to an equine in multiple applications at more than one location on the equine's skin. In another embodiment, the single dose transdermal formulation of the composition can be transdermally administered to an equine in multiple applications at the same location on the equine's skin.

In some embodiments, the single dose transdermal formulation of the composition comprises between about 0.1 and about 10 mL of a solution of the composition. In some embodiments, the single dose transdermal formulation of the composition comprises between about 0.2 and about 8 mL of a solution of the composition. In some embodiments, the single dose transdermal formulation of the composition comprises between about 0.3 and about 6 mL of a solution of the composition. In some embodiments, the single dose transdermal formulation of the composition comprises between about 0.5 and about 5 mL of a solution of the composition. In some embodiments, the single dose transdermal formulation of the composition comprises between about 0.7 and about 3 mL of a solution of the composition. In some embodiments, the single dose transdermal formulation of the composition comprises between about 0.8 and about 2 mL of a solution of the composition. In one embodiment, the single dose transdermal formulation of the composition comprises about 0.9 mL of a solution of the composition. In another embodiment, the single dose transdermal formulation of the composition comprises about 1.0 mL of a solution of the composition.

In one embodiment, a single dose transdermal formulation of the composition is transdermally administered to an equine in a single application at one location on the equine's skin, wherein the single application is up to about 0.9 mL of a solution of the composition. In another embodiment, a single dose transdermal formulation of the composition is transdermally administered to an equine in multiple applications at the same location on the equine's skin, wherein each application is up to about 0.9 mL of a solution of the composition. In yet another embodiment, a single dose transdermal formulation of the composition is transdermally administered to an equine in multiple applications at more than one location on the equine's skin, wherein each application is up to about 0.9 mL of a solution of the composition. In embodiments wherein multiple applications of the composition are utilized, the multiple applications can be administered to the equine over a reasonable duration of time.

### EXAMPLE 1

### Comparative Pharmacokinetic Study of a Transdermal Fentanyl Composition Administered to Equines and Canines

A study of the methods of the present invention can be undertaken to compare pharmacokinetic parameters of the composition of the present invention following its transdermal administration. For example, the pharmacokinetic parameters of fentanyl administered via the composition of the present invention can be evaluated in equines and canines.

The composition of the present invention can be transdermally administered to equines at a dose of about 0.04 mg/kg. The composition can be transdermally administered to the clipped and clean skin of the foreleg of the equines. Following administration to the equines, the maximum mean plasma concentration of fentanyl was observed approximately five hours post-administration and was calculated to be approximately 9 ng/ml. The maximum mean plasma concentration of fentanyl observed in equines was at least four times greater than the targeted therapeutic concentration (*i.e.,* about 1 ng/ml) observed in canines and in humans.

Following transdermal administration of the composition of the present invention, the duration of time required to achieve a therapeutic plasma concentration of fentanyl in equines was less than one hour. Furthermore, plasma concentrations of fentanyl in equines remained above the therapeutic concentration threshold for approximately 12 hours. At approximately 24 hours after administration of the composition of the present invention, the average plasma concentrations of fentanyl in equines decreased to approximately 0.116 ng/ml. The administered doses of the composition of the present invention were well tolerated in equines and no abnormal application site reactions were observed. In addition, equines administered doses of the composition did not demonstrate excessive CNS excitation.

Pharmacokinetic parameters of fentanyl following transdermal administration of the composition of the present invention are summarized in Table 1. Pharmacokinetic parameters of fentanyl in equines administered the composition of the present invention are unique compared to pharmacokinetic parameters in canines administered the composition of the present invention. For example, the amount of fentanyl absorbed into the systemic circulation of equines following transdermal administration of the composition is unexpectedly higher than anticipated based on comparative allometric calculations of fentanyl absorption in canines. Furthermore, significantly higher doses of transdermally administered fentanyl are necessary to obtain a fentanyl plasma concentration of at least 1 ng/ml in canines.

**Table 1. Pharmacokinetics of fentanyl following transdermal application of the composition in equines and canines**

| **Species** | **Dose (mg/kg)** | **Average Cmax (ng/ml)** | **Average half-life (hours)** | **Time to attain plasma concentrations > 1 ng/ml (hours)** | **Duration of time with concentrations > 1 ng/ml (hours)** | **Total fentanyl dose applied (mg)** |
|---|---|---|---|---|---|---|
| Equine | 0.04 | 9 | 26.9 | <1 | 12 | 18 |
| Canine | 2.6 | 2.67 | 71.96 | 8 to 24 | 60-168 | 26 |

Various studies of fentanyl administration via a transdermal patch formulation indicate that the therapeutically effective dose of fentanyl is lower in equines compared to canines. A summary of these data is shown in Table 2.

**Table 2. Therapeutically effective doses of fentanyl following administration via transdermal patches (Duragesic®)***

| **Species** | **Fentanyl concentration per patch (ug/hour)** | **No. of patches administered** | **Average amount of fentanyl administered (mg)** | **Average weight of animal (kg)** | **Total fentanyl dose administered (mg/kg)** |
|---|---|---|---|---|---|
| Equine | 100 | 2 | 20 | 425 | 0.047 |
| Canine | 50 | 1 | 5 | 15 | 0.33 |

| | | | | | |
|---|---|---|---|---|---|
| *Source: Plumb's Veterinary Drug Handbook, 6th edition, 2000, Blackwell Publishing, Ames, Iowa, p. 381. | | | | | |

Furthermore, transdermal administration of the composition of the present invention results in an unexpectedly higher absorption of fentanyl in equines compared to the anticipated amount of absorption estimated from administration of fentanyl via a transdermal patch. For example, the therapeutically effective dose of fentanyl transdermally administered to equines via a transdermal patch is approximately 7-fold less than the therapeutically effective dose of fentanyl in canines. In contrast, the therapeutically effective dose of fentanyl transdermally administered to equines via the composition of the present invention is approximately 70-fold less than the therapeutically effective dose of fentanyl in canines. These results suggest that the absorption of fentanyl following transdermal administration of the composition of the present invention to equines is unique and unexpected based on comparative data of fentanyl transdermally administered to canines via a transdermal patch.

In addition, transdermal administration of the composition of the present invention to equines achieves less variability of fentanyl pharmacokinetic parameters compared to administration of fentanyl to equines via a transdermal patch. A summary of these data is shown in Table 3.

**Table 3. Comparison of fentanyl pharmacokinetic parameters in equines following transdermal application of the composition or a transdermal patch**

| **Species** | **Application Form** | **Dosage (mg/kg)** | **Average Cmax (ng/mL)** | **Average half-life (hours)** |
|---|---|---|---|---|
| Equine | Transdermal solution | 0.033-0.043 | 2.6 | 8 |
| Equine* | Transdermal patch | 0.06-0.067 | 0.67-5.12 | 8.5-14.5 |

| | | | | |
|---|---|---|---|---|
| *Source: Orsini, "Pharmacokinetics of fentanyl delivered transdermally in healthy adult horses - variability among horses and its clinical implications," J of Veterinary Pharmacology and Therapeutics, 2006; 29:539-546. | | | | |

## Claims

1. A transdermal solution composition comprising fentanyl, octyl salicylate, and a volatile liquid, in a therapeutically effective amount, for use in controlling pain in an equine in need thereof for an effective period of time by transdermal administration, wherein the fentanyl is administered at a dose of 0.01 to 0.1 mg/kg of weight of the equine.

2. The composition for use according to claim 1, wherein the effective period of time is about 12 hours.

3. The composition for use according to claim 1 or claim 2, wherein the composition is administered as a single dose.

4. The composition for use according to any one of claims 1 to 3, wherein the volatile liquid is isopropanol.

5. The composition for use according to any one of claims 1 to 4, wherein the pain is associated with colic.

6. The composition for use according to any one of claims 1 to 5, wherein the pain is associated with a surgery performed or to be performed on the equine.

7. The composition for use according to any one of claims 1 to 6, wherein the composition comprises 0.1 to 10% (w/v) of fentanyl, 0.1 to 10% (w/v) of the penetration enhancer, and 80 to 99.8% (w/v) of the volatile liquid.

8. The composition for use according to any one of claims 1 to 7, wherein the composition is administered with one or more other therapeutic ingredients.

9. The composition for use according to any one of claims 1 to 8, wherein the composition is administered using a transdermal dispensing apparatus.

## Patentansprüche

1. Transdermale Lösungszusammensetzung, die Fentanyl, Octylsalicylat und eine flüchtige Flüssigkeit enthält, in einer therapeutisch wirksamen Menge zur Verwendung bei der Kontrolle von Schmerzen bei einem Pferd, das diese für einen effektiven Zeitraum benötigt, durch transdermale Verabreichung , wobei das Fentanyl in einer Dosis von 0,01 bis 0,1 mg/kg des Gewichts des Pferdes verabreicht wird.

2. Zusammensetzung zur Verwendung nach Anspruch 1, wobei die effektive Zeitspanne etwa 12 Stunden beträgt.

3. Zusammensetzung zur Verwendung nach Anspruch 1 oder Anspruch 2, wobei die Zusammensetzung als Einzeldosis verabreicht wird.

4. Zusammensetzung zur Verwendung nach einem der Ansprüche 1 bis 3, wobei die flüchtige Flüssigkeit Isopropanol ist.

5. Zusammensetzung zur Verwendung nach einem der Ansprüche 1 bis 4, wobei der Schmerz mit Kolik verbunden ist.

6. Zusammensetzung zur Verwendung nach einem der Ansprüche 1 bis 5, wobei der Schmerz mit einer Operation verbunden ist, die am Pferd durchgeführt wird oder durchgeführt werden soll.

7. Zusammensetzung zur Verwendung nach einem der Ansprüche 1 bis 6, wobei die Zusammensetzung 0,1 bis 10% (w/v) Fentanyl, 0,1 bis 10% (w/v) des Penetrationsverstärkers und 80 bis 99,8% (w/v) der flüchtigen Flüssigkeit umfasst.

8. Zusammensetzung zur Verwendung nach einem der Ansprüche 1 bis 7, wobei die Zusammensetzung mit einem oder mehreren anderen therapeutischen Inhaltsstoffen verabreicht wird.

9. Zusammensetzung zur Verwendung nach einem der Ansprüche 1 bis 8, wobei die Zusammensetzung unter Verwendung einer transdermalen Ausgabevorrichtung verabreicht wird.

## Revendications

1. Composition en solution à usage transdermique comprenant du fentanyl, du salicylate d'octyle, et un liquide volatil, en une quantité efficace du point de vue thérapeutique, destinée à être utilisée dans le contrôle de la douleur chez un équidé en ayant besoin pendant une période de temps efficace par administration transdermique, dans laquelle le fentanyl est administré à une dose de 0,01 à 0,1 mg/kg de poids de l'équidé.

2. Composition destinée à être utilisée selon la revendication 1, dans laquelle la période de temps efficace est d'environ 12 heures.

3. Composition destinée à être utilisée selon la revendication 1 ou la revendication 2, dans laquelle la composition est administrée en une seule dose.

4. Composition destinée à être utilisée selon l'une quelconque des revendications 1 à 3, dans laquelle le liquide volatil est l'isopropanol.

5. Composition destinée à être utilisée selon l'une quelconque des revendications 1 à 4, dans laquelle la douleur est associée à une colique.

6. Composition destinée à être utilisée selon l'une quelconque des revendications 1 à 5, dans laquelle la douleur est associée à une opération chirurgicale effectuée ou devant être effectuée sur l'équidé.

7. Composition destinée à être utilisée selon l'une quelconque des revendications 1 à 6, dans laquelle la composition comprend 0,1 à 10 % (p/v) de fentanyl, 0,1 à 10 % (p/v) de l'amplificateur de pénétration, et 80 à 99,8 % (p/v) du liquide volatil.

8. Composition destinée à être utilisée selon l'une quelconque des revendications 1 à 7, dans laquelle la composition est administrée avec un ou plusieurs autres ingrédients thérapeutiques.

9. Composition destinée à être utilisée selon l'une quelconque des revendications 1 à 8, dans laquelle la composition est administrée au moyen d'un dispositif de délivrance transdermique.
